(19) Europäisches Patentamt
European Patent Office
Office européen des brevets

(11) EP 4 647 423 A1

(12) **EUROPEAN PATENT APPLICATION**
published in accordance with Art. 153(4) EPC

(43) Date of publication:
**12.11.2025 Bulletin 2025/46**

(21) Application number: **23914174.0**

(22) Date of filing: **14.04.2023**

(51) International Patent Classification (IPC):
***C07D 211/46*** (2006.01) ***A61P 37/08*** (2006.01)
***A61P 11/02*** (2006.01) ***A61P 17/00*** (2006.01)
***A61P 17/04*** (2006.01) ***A61K 31/445*** (2006.01)

(86) International application number:
**PCT/CN2023/088514**

(87) International publication number:
**WO 2024/146022 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.01.2023 CN 202310000722**

(71) Applicant: **Chengdu Shibeikang Biomedical Technology Co., Ltd.**
**Chengdu, Sichuan 611731 (CN)**

(72) Inventors:
- **WANG, Xiaoyu**
  **Chengdu, Sichuan 611730 (CN)**
- **ZHANG, Li**
  **Chengdu, Sichuan 611730 (CN)**
- **JIANG, Jie**
  **Chengdu, Sichuan 611730 (CN)**
- **ZHOU, Ning**
  **Chengdu, Sichuan 611730 (CN)**
- **MOU, Xia**
  **Chengdu, Sichuan 611730 (CN)**

(74) Representative: **Herrero & Asociados, S.L.**
**Edificio Aqua - Agustín de Foxá, 4-10**
**28036 Madrid (ES)**

(54) **CRYSTAL FORM AND AMORPHOUS FORM OF CAREBASTINE P-TOLUENESULFONATE**

(57) Provided are a crystal form and amorphous form of carebastine p-toluenesulfonate, and a preparation method therefor, relating to the field of medicinal chemistry, for use in solving the problems in the prior art that carebastine is unstable, not easy to scale up for synthesis, and difficult to make into a drug and the like. The crystal form and amorphous form of carebastine p-toluenesulfonate have the characteristics of high purity, high stability, high bioavailability, easy scale-up synthesis, and industrial production and the like, and have excellent hygroscopicity for easy storage.

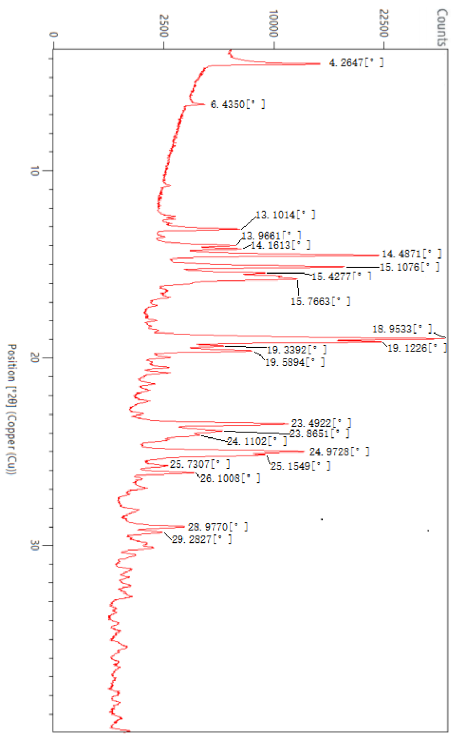

FIG 1



EP 4 647 423 A1

## Description

This application claims priority to the patent for invention with Patent No. CN202310000722.6, entitled "Crystal form and amorphous form of Carebastine p-toluenesulfonate", filed on January 03, 2023, which is hereby expressly incorporated by reference herein.

## Technical Field

The invention relates to the technical field of pharmaceutical chemistry, in particular to a crystal form and an amorphous form of carebastine p-toluenesulfonate and application and preparation method thereof.

## Background

Carebastine is an active metabolite of the second-generation histamine H1 receptor antagonist ebastine, and though neither enters the central system and has any adverse central nervous effect or anti-cholinergic effect, carebastine can also avoid the liver first-pass effect of ebastine, takes effect quickly, and can be used for the treatment of allergic diseases such as urticaria, allergic rhinitis, eczema, dermatitis, prurigo, skin pruritus and the like, particularly for the emergency treatment of acute allergic diseases such as acute urticaria, asthma and the like. However, carebastine has the defects of light instability, difficulty in purification, difficulty in scaled-up synthesis, difficulty in pharmaceutical formulation and the like, and thus carebastine is not commercially available on the market at present. In addition, there are no detailed reports on salts of carebastine, and there is no reported carebastine p-toluenesulfonate and crystal form or amorphous form thereof. The present invention aims to provide a crystal form and an amorphous form of carebastine p-toluenesulfonate to overcome the defects in the prior art and provide a clinically reliable option.

## Summary of the Invention

In order to solve at least one of the technical problems existing in the prior art, the present invention provides a crystal form and an amorphous form of carebastine p-toluenesulfonate. The crystal form and the amorphous form of carebastine p-toluenesulfonate of the present invention have high stability, good hygroscopicity and high bioavailability, and are easy to purify, suitable for scaled-up production, and favorable for pharmaceutical formulation.

In one aspect, the present invention provides a crystal form of carebastine p-toluenesulfonate represented by formula (1),

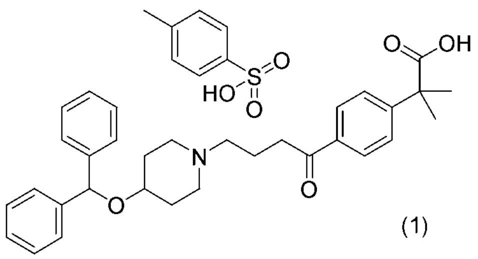

(1)

wherein the crystal form has an X-ray powder diffraction pattern obtained by Cu-Kα radiation that has diffraction peaks at 2θ positions of: 4.26±0.2°, 6.43±0.2°, 14.49±0.2°, 15.11±0.2°, and 18.95±0.2°.

Further, the crystal form has an X-ray powder diffraction pattern that has diffraction peaks at 2θ positions of: 4.26 ±0.2°, 6.43±0.2°, 14.49±0.2°, 15.11±0.2°, 18.95±0.2°, and optionally 19.12±0.2°.

Further, the crystal form has an X-ray powder diffraction pattern that has diffraction peaks at 2θ positions of: 4.26 ±0.2°, 6.43±0.2°, 13.10±0.2°, 14.49±0.2°, 15.11±0.2°, 15.77±0.2°, and 18.95±0.2°。

Preferably, the crystal form has an X-ray powder diffraction pattern that has diffraction peaks at 2θ positions of: 4.26±0.2°, 6.43±0.2°, 13.10±0.2°, 14.49±0.2°, 15.11±0.2°, 15.77±0.2°, 18.95±0.2°, and 19.12±0.2°.

Further, the crystal form has an X-ray powder diffraction pattern that has diffraction peaks at 2θ positions of: 4.26 ±0.2°, 6.43±0.2°, 13.10±0.2°, 14.49±0.2°, 15.11±0.2°, 15.77±0.2°, 18.95±0.2°, 19.59±0.2°, 23.49±0.2°, and 24.97 ±0.2°。

Preferably, the crystal form has an X-ray powder diffraction pattern that has diffraction peaks at 2θ positions of: 4.26±0.2°, 6.43±0.2°, 13.10±0.2°, 14.49±0.2°, 15.11±0.2°, 15.77±0.2°, 18.95±0.2°, 19.12±0.2°, 19.59±0.2°, 23.49 ±0.2°, and 24.97±0.2°.

Further, the crystal form has an X-ray powder diffraction pattern that has diffraction peaks at 2θ positions of: 4.26 ±0.2°, 6.43±0.2°, 13.10±0.2°, 13.97±0.2°, 14.16±0.2°, 14.49±0.2°, 15.11±0.2°, 15.43±0.2°, 15.77±0.2°, 18.95±0.2°, 19.12±0.2°, 19.34±0.2°, 19.59±0.2°, 23.49±0.2°, 23.87±0.2°, 24.11±0.2°, 24.97±0.2°, 25.15±0.2°, 25.73 ±0.2°, 26.10±0.2°, 28.98±0.2°, and 29.28±0.2°.

Preferably, the crystal form has an X-ray powder diffraction pattern that has diffraction peaks at 2θ positions of: 4.26±0.2°, 6.43±0.2°, 13.10±0.2°, 13.97±0.2°, 14.16±0.2°, 14.49±0.2°, 15.11±0.2°, 15.43±0.2°, 15.61±0.2°, 15.77 ±0.2°, 18.95±0.2°, 19.12±0.2°, 19.34±0.2°, 19.59±0.2°, 23.49±0.2°, 23.87±0.2°, 24.11±0.2°, 24.97±0.2°, 25.15 ±0.2°, 25.73±0.2°, 26.10±0.2°, 28.98±0.2°, and 29.28±0.2°.

Further, the crystal form has an X-ray powder diffraction pattern that has diffraction peaks at 2θ positions of: 4.26 ±0.2°, 6.43±0.2°, 10.77±0.2°, 12.41±0.2°, 12.56±0.2°, 12.78±0.2°, 13.10±0.2°, 13.97±0.2°, 14.16±0.2°, 14.49 ±0.2°, 15.11±0.2°, 15.43±0.2°, 15.77±0.2°, 18.95±0.2°, 19.12±0.2°, 19.34±0.2°, 19.59±0.2°, 23.49±0.2°, 23.87 ±0.2°, 24.11±0.2°, 24.97±0.2°, 25.15±0.2°, 25.73±0.2°, 26.10±0.2°, 28.98±0.2°, and 29.28±0.2°.

Preferably, the crystal form has an X-ray powder diffraction pattern that has diffraction peaks at 2θ positions of: 4.26±0.2°, 6.43±0.2°, 10.77±0.2°, 12.41±0.2°, 12.56±0.2°, 12.78±0.2°, 13.10±0.2°, 13.97±0.2°, 14.16±0.2°, 14.49 ±0.2°, 15.11±0.2°, 15.43±0.2°, 15.61±0.2°, 15.77±0.2°, 18.95±0.2°, 19.12±0.2°, 19.34±0.2°, 19.59±0.2°, 23.49 ±0.2°, 23.87±0.2°, 24.11±0.2°, 24.97±0.2°, 25.15±0.2°, 25.73±0.2°, 26.10±0.2°, 28.98±0.2°, and 29.28±0.2°.

Further, the crystal form has an X-ray powder diffraction pattern with characteristic peaks at the following 2θ positions and corresponding relative intensities:

| 2θ | Relative intensity ($I/I_0$) |
|---|---|
| 4.26±0.2° | 28.8±5% |
| 6.43±0.2° | 3.7±5% |
| 14.49±0.2° | 65.1±5% |
| 15.11±0.2° | 53.8±5% |
| 18.95±0.2° | 100±5% |
| Optionally 19.12±0.2° | 67.1±5% |

Preferably, the crystal form has an X-ray powder diffraction pattern with characteristic peaks at the following 2θ positions and corresponding relative intensities:

| 2θ | Relative intensity ($I/I_0$) |
|---|---|
| 4.26±0.2° | 28.8% |
| 6.43±0.2° | 3.7% |
| 14.49±0.2° | 65.1% |
| 15.11±0.2° | 53.8% |
| 18.95±0.2° | 100% |
| Optionally 19.12±0.2° | 67.1% |

Further, the crystal form has an X-ray powder diffraction pattern with characteristic peaks at the following 2θ positions and corresponding relative intensities:

| 2θ | Relative intensity ($I/I_0$) |
|---|---|
| 4.26±0.2° | 28.8±5% |
| 6.43±0.2° | 3.7±5% |
| Optionally 13.10±0.2° | 16.3±5% |
| 14.49±0.2° | 65.1±5% |
| 15.11±0.2° | 53.8±5% |
| Optionally 15.77±0.2° | 30.9±5% |
| 18.95±0.2° | 100±5% |

(continued)

| 2θ | Relative intensity ($I/I_0$) |
|---|---|
| Optionally 19.12±0.2° | 67.1±5% |
| Optionally 19.59±0.2° | 21.5±5% |
| Optionally 23.49±0.2° | 38.8±5% |
| Optionally 24.97±0.2° | 42.8±5% |

Preferably, the crystal form has an X-ray powder diffraction pattern with characteristic peaks at the following 2θ positions and corresponding relative intensities:

| 2θ | Relative intensity ($I/I_0$) |
|---|---|
| 4.26±0.2° | 28.8% |
| 6.43±0.2° | 3.7% |
| Optionally 13.10±0.2° | 16.3% |
| 14.49±0.2° | 65.1% |
| 15.11±0.2° | 53.8% |
| Optionally 15.77±0.2° | 30.9% |
| 18.95±0.2° | 100% |
| Optionally 19.12±0.2° | 67.1% |
| Optionally 19.59±0.2° | 21.5% |
| Optionally 23.49±0.2° | 38.8% |
| Optionally 24.97±0.2° | 42.8% |

Furthermore, the crystal form has an X-ray powder diffraction pattern substantially as shown in FIG. 1.

It is noteworthy that, in the X-ray powder diffraction pattern of the above crystal form, there may be an insignificant spike peak due to the difference in the detection conditions such as the degree of powder grinding, or a phenomenon of the presence of a flat peak or slight bulge caused by merging of a larger peak, at 2θ positions such as 15.61±0.2°, 19.12±0.2° and 25.15±0.2°, and it should be understood that a small difference in the peak morphology at these peak positions shall be within the scope of the present invention.

Further, the crystal form has a differential scanning calorimetry thermogram having an endothermic peak at 171°C±5°C.

Preferably, the crystal form has a differential scanning calorimetry thermogram having an endothermic peak at 171.51°C±3°C.

Further preferably, the crystal form has a differential scanning calorimetry thermogram having an endothermic peak at 171.51°C±1.0°C.

Furthermore, the crystal form has a differential scanning calorimetry curve substantially as shown in FIG. 2.

Further, the crystal form has a thermogravimetric analysis graph substantially as shown in FIG. 3.

In another aspect, the present invention provides an amorphous form of carebastine p-toluenesulfonate represented by formula (1),

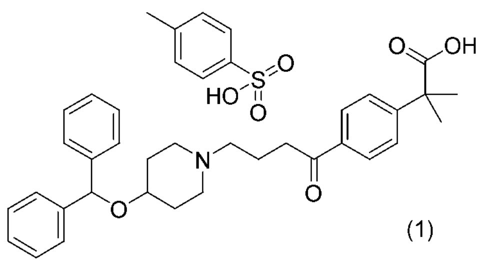

(1)

.

Further, the amorphous form has an X-ray powder diffraction pattern obtained by Cu-Kα radiation which is represented by a diffuse peak; preferably, the amorphous form has an X-ray powder diffraction pattern substantially as shown in FIG. 5.

In a third aspect, the present invention provides a method for preparing any of the crystal form or any of the amorphous form as described above, comprising a salt-forming step of: dissolving an appropriate amount of 2-(4-(4-(4-(diphenylmethoxy)piperidin-1-yl)butyryl)phenyl)-2-methylpropanoic acid and p-toluenesulfonic acid in an organic solvent under stirring and heating to conduct a reaction to form a salt, lowering the temperature to precipitate a solid after completion of the reaction, and suction filtering to obtain a crude product of carebastine p-toluenesulfonate.

Further, the organic solvent used in the above salt-forming step includes, but is not limited to, alcohols, esters, ethers, ketones, aromatic hydrocarbons, glycol derivatives, pyrrolidones, tetrahydrofurans, amides, or other types.

Further, the alcohols include C1-C6 straight or branched saturated alkyl alcohols, preferably methanol, ethanol, propanol or isopropanol; the esters include lower alkyl esters, preferably ethyl acetate, propyl acetate or isopropyl acetate; the ethers comprise lower alkyl ethers, preferably methyl tert-butyl ether or ethyl ether; the ketones include lower alkanones, preferably acetone or butanone; the aromatic hydrocarbons include benzene, toluene, xylene or mesitylene, preferably toluene; the glycol derivatives include ethylene glycol monomethyl ether, ethylene glycol monoethyl ether or ethylene glycol monobutyl ether, preferably ethylene glycol monomethyl ether; the pyrrolidinones include N-methyl pyrrolidone or N-ethyl pyrrolidone; the tetrahydrofurans comprise tetrahydrofuran or 2-methyltetrahydrofuran; the amides include N,N-dimethylformamide or N,N-dimethylacetamide; and the other types include dioxane, acetonitrile, dimethyl sulfoxide, or a mixture of the organic solvent and water.

Further preferably, the organic solvent includes methanol, ethanol, isopropanol, acetone, butanone, acetonitrile, ethyl acetate, isopropyl acetate, toluene, tetrahydrofuran, dioxane, N,N-dimethylformamide, N,N-dimethylacetamide, acetonitrile, N-methylpyrrolidinone, methyl tert-butyl ether, ethyl ether, dimethyl sulfoxide, or a mixture of the organic solvent and water.

Furthermore, the organic solvent comprises ethanol, butanone, acetonitrile or isopropyl acetate.

Further, the reaction temperature of the salt-forming step is 40 to 100°C; preferably 60 to 90°C.

Further, the solid precipitation temperature of the salt-forming step is -20 to 30°C; preferably -5 to 20°C.

Further, the present invention provides a method for preparing the crystal form as described above, comprising the following steps: adding the crude product of carebastine p-toluenesulfonate into a solvent, heating and stirring, lowering the temperature to precipitate a crystal, suction filtering and drying to obtain a crystal of carebastine p-toluenesulfonate.

Further, the solvent used in the method for preparing the crystal form includes one or more of water, alcohols, esters, ethers, ketones, aromatic hydrocarbons, glycol derivatives, pyrrolidones, tetrahydrofurans, amides and other types of organic solvents.

Further, the alcohols include C1-C6 straight or branched saturated alkyl alcohols, preferably methanol, ethanol, propanol or isopropanol; the esters include lower alkyl esters, preferably ethyl acetate, propyl acetate or isopropyl acetate; the ethers comprise lower alkyl ethers, preferably methyl tert-butyl ether or ethyl ether; the ketones include lower alkanones, preferably acetone or butanone; the aromatic hydrocarbons include benzene, toluene, xylene or mesitylene, preferably toluene; the glycol derivatives include ethylene glycol monomethyl ether, ethylene glycol monoethyl ether or ethylene glycol monobutyl ether, preferably ethylene glycol monomethyl ether; the pyrrolidinones include N-methyl pyrrolidone or N-ethyl pyrrolidone; the tetrahydrofurans comprise tetrahydrofuran or 2-methyltetrahydrofuran; the amides include N,N-dimethylformamide or N,N-dimethylacetamide; and the other types of organic solvents include dioxane, acetonitrile or dimethyl sulfoxide.

Further preferably, the solvent used in the method for preparing the crystal form includes one or more of water, methanol, ethanol, isopropanol, ethyl acetate, isopropyl acetate, methyl tert-butyl ether, acetone, butanone, toluene, ethylene glycol monomethyl ether, N-methylpyrrolidone, N-ethylpyrrolidone, tetrahydrofuran, N,N-dimethylformamide, N,N-dimethylacetamide, dioxane, acetonitrile, and dimethyl sulfoxide.

More preferably, the solvent used in the method for preparing the crystal form includes one or more of water, methanol, ethanol, isopropanol, ethyl acetate, isopropyl acetate, methyl tert-butyl ether, acetone, butanone, toluene, N-methylpyrrolidone, N-ethylpyrrolidone, tetrahydrofuran, N,N-dimethylformamide, N,N-dimethylacetamide, dioxane, acetonitrile, and dimethyl sulfoxide.

In particular, when a composite solvent is used, a mixture of the organic solvent and water is preferred.

Further, the reaction temperature of the method for preparing the crystal form is 30 to 100°C; preferably 50 to 90°C.

Further, the crystal precipitation temperature of the method for preparing the crystal form is - 20 to 35°C; preferably -5 to 25°C; more preferably 0 to 20°C.

Further, the method for preparing the amorphous form as described above comprises the following steps: adding the crude product of carebastine p-toluenesulfonate into a solvent, heating and refluxing, suction filtering after the solid is completely dissolved, and spray drying or lyophilizing the filtrate to obtain a white solid.

Further, the solvent used in the method for preparing the amorphous form includes one or more of water, alcohols, esters, ethers, ketones, aromatic hydrocarbons, glycol derivatives, pyrrolidones, tetrahydrofurans, amides and other types of organic solvents.

Further, the alcohols include C1-C6 straight or branched saturated alkyl alcohols, preferably methanol, ethanol, propanol or isopropanol; the esters comprise lower alkyl esters, preferably ethyl acetate, propyl acetate or isopropyl acetate; the ethers include lower alkyl ethers, preferably methyl tert-butyl ether or ethyl ether; the ketones include lower alkanones, preferably acetone or butanone; the aromatic hydrocarbons include benzene, toluene, xylene or mesitylene, preferably toluene; the glycol derivatives include ethylene glycol monomethyl ether, ethylene glycol monoethyl ether or ethylene glycol monobutyl ether, preferably ethylene glycol monomethyl ether; the pyrrolidinones include N-methyl pyrrolidone or N-ethyl pyrrolidone; the tetrahydrofurans comprise tetrahydrofuran or 2-methyltetrahydrofuran; the amides include N,N-dimethylformamide or N,N-dimethylacetamide; and the other types of organic solvents include dioxane, acetonitrile or dimethyl sulfoxide.

Further preferably, the solvent used in the method for preparing the amorphous form includes one or more of water, methanol, ethanol, isopropanol, ethyl acetate, isopropyl acetate, methyl tert-butyl ether, acetone, butanone, toluene, ethylene glycol monomethyl ether, N-methylpyrrolidone, N-ethylpyrrolidone, tetrahydrofuran, N,N-dimethylformamide, N,N-dimethylacetamide, dioxane, acetonitrile, and dimethyl sulfoxide.

More preferably, the solvent used in the method for preparing the amorphous form includes one or two of water, methanol, ethanol, isopropanol, ethyl acetate, isopropyl acetate, methyl tert-butyl ether, acetone, butanone, toluene, N-methylpyrrolidone, N-ethylpyrrolidone, tetrahydrofuran, N,N-dimethylformamide, N,N-dimethylacetamide, dioxane, acetonitrile, and dimethyl sulfoxide.

In particular, when a composite solvent is used, a mixture of the organic solvent and water is preferred.

Further, the reflux temperature of the method for preparing the amorphous form is 30 to 100°C; preferably 50 to 90°C.

In a fourth aspect, the present invention further provides a pharmaceutical composition comprising any of the crystal form or the amorphous form of carebastine p-toluenesulfonate as described above, wherein the pharmaceutical composition further comprises a pharmaceutically acceptable carrier.

The "pharmaceutically acceptable carrier" refers to a diluent, adjuvant, excipient or medium that is administered together with an active ingredient and that is suitable for contacting human and/or other animal tissues without excessive toxicity, irritation, allergic response or other problems or complications corresponding to a reasonable benefit/risk ratio within reasonable medical judgment.

In a fifth aspect, the present invention further provides use of any of the crystal form or amorphous form of carebastine p-toluenesulfonate as described above in the manufacture of a medicament of a histamine H1 receptor antagonist.

Further, the use comprises use in the manufacture of a medicament for treating and/or preventing an allergic disease.

Further, the allergic disease further includes an acute allergic disease.

Further, the allergic disease is selected from urticaria, allergic rhinitis, eczema, dermatitis and skin pruritus; preferably, the acute allergic disease is selected from acute urticaria and acute allergic rhinitis.

It should be noted that, unless there is evidence to the contrary or specifically stated, the compound represented by formula (1) of the present invention indicates that the molar ratio of carebastine to p-toluenesulfonic acid is 1:1, and the molar ratio thereof is also 1:1 in the corresponding crystal form and amorphous form.

The solvent used in the methods for preparing the crystal form and amorphous form of the present invention comprises "a mixture of the organic solvent and water", wherein the term "the organic solvent" includes, but is not limited to, all the organic solvents listed in the present invention.

The term "crude product" of the present invention means that the product has a purity of no more than 99%, preferably no more than 98.5%.

Advantageous effect

Compared with the prior art, the present invention has the following advantageous effects: The crystal form and amorphous form of carebastine p-toluenesulfonate of the present invention: (1) have good pharmaceutical solid morphology, no or little hygroscopicity, high purity and stability, and are convenient for transportation and storage; (2) have a simple production process, with inexpensive and readily available materials, can be easily scaled up, have a short production cycle, and are energy efficient and environmentally friendly; and (3) have better pharmacokinetic properties, higher blood concentration, faster onset of action, longer half-life, and possess the potential to be used in the treatment of acute allergic diseases such as acute urticaria, with stronger and longer-lasting efficacy and higher safety.

**Brief Description of Drawings**

FIG. 1 is an X-ray powder diffraction (XRPD) pattern of a crystal form of carebastine p-toluenesulfonate;

FIG. 2 is a differential scanning calorimetry (DSC) thermogram of a crystal form of carebastine p-toluenesulfonate;

FIG. 3 is a thermogravimetric analysis (TGA) graph of a crystal form of carebastine p-toluenesulfonate;

FIG. 4 is a stacked XRPD pattern of the crystal forms of carebastine p-toluenesulfonate of Examples 2 to 6;

FIG. 5 is an X-ray powder diffraction (XRPD) pattern of an amorphous form of carebastine p-toluenesulfonate.

**Detailed Description of the Invention**

The present invention will be further described in detail below with reference to embodiments and test examples, and the embodiments and test examples of the present invention are only used to illustrate the technical solutions of the present invention and are not intended to limit the present invention. Any equivalent replacement in the field made in accordance with the disclosure of the present invention falls within the protection scope of the present invention.

The structure of the compound was determined by nuclear magnetic resonance ($^1$H NMR) or liquid chromatography-mass spectrometry (LC-MS).

The instrument for liquid chromatography-mass spectrometer (LC-MS) is Agilent G6120B (together with liquid chromatography Agilent 1260); the nuclear magnetic resonance instrument ($^1$H NMR) is Bruker AVANCE-400 or Bruker AVANCE-800, wherein the nuclear magnetic resonance ($^1$H NMR) shift ($\delta$) is given in a unit of parts per million (ppm), with $CDCl_3$ as the measured solvent and tetramethylsilane (TMS) as an internal standard; and the chemical shift is given in an unit of $10^{-6}$ (ppm).

The term "room temperature" in the present invention refers to a temperature between 10°C and 30°C.

In the following examples and test examples, the ebastine (Comparative Example 1) used is in a free form, commercially available, with a purity of 98% or more; and the carebastine (Comparative Example 2) used is in a free form, commercially available, with a purity of 99% or more.

**Example 1:** Preparation of crude 2-(4-(4-(4-(diphenylmethoxy)piperidine-1-yl)butyryl)phenyl)-2-methylpropanoic acid p-toluenesulfonate (carebastine p-toluenesulfonate)

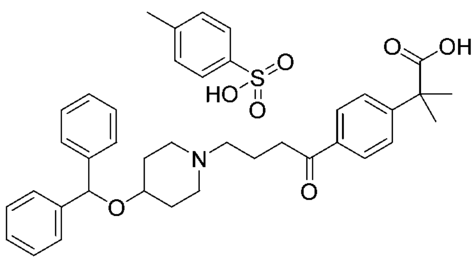

The synthesis route is as follows:

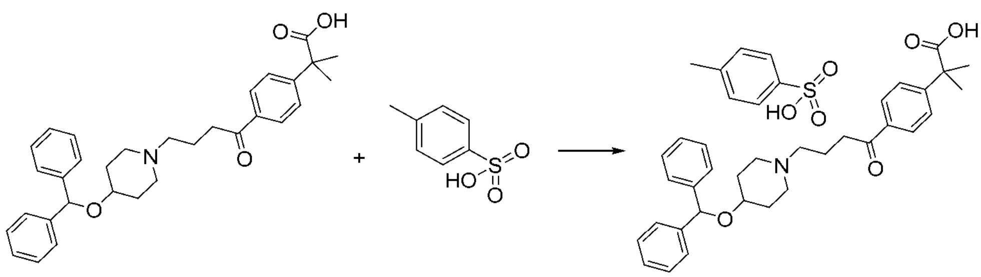

To a three-necked flask, 2-(4-(4-(4-(diphenylmethoxy)piperidine-1-yl)butyryl)phenyl)-2-methylpropanoic acid (carebastine) (500 g, 0.1 mol) and 1 L of isopropanol were added and heated to 60-90°C, and p-toluenesulfonic acid (172 g, 1 mol) was added thereto under stirring. The temperature was lowered, and a white solid was precipitated, filtered and dried, to obtain 571 g of carebastine p-toluenesulfonate, with a yield of 85% and a purity of 98.05%.

ESI-MS: m/z = 500.3(M+H) $^+$.

$^1$H NMR (600 MHz, $CDCl_3$) δ: 10.01 (s, 1H), 7.80~7.83 (d, 2H), 7.74~7.76 (d, 2H), 7.43~7.46(d, 2H), 7.27~7.37 (m, 10H), 7.13~7.15 (d, 2H), 5.44 (s, 1H), 3.83~3.85 (m, 1H), 3.53~3.55 (m, 2H), 3.10~3.17 (m, 4H), 3.00~3.05(m, 2H), 2.32(s, 3H), 2.21~2.29 (m, 2H), 2.09~2.19 (m, 2H), 2.01~2.08 (m, 2H), 1.58 (s, 6H).

**Example** 2: Preparation of a crystal form of carebastine p-toluenesulfonate

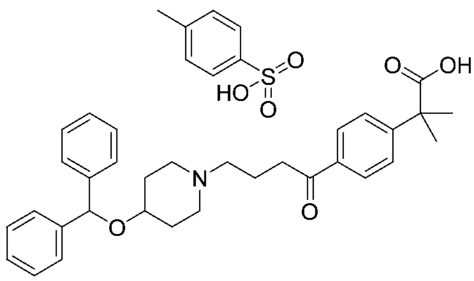

1.5 kg of ethanol and 100 g of crude carebastine p-toluenesulfonate were added at room temperature, and the temperature was raised under stirring to 60-80°C and kept for 0.5-1.2 h under stirring. The temperature was then lowered to 5-20°C, and crystals were precipitated for 0.5-1.5 h. After filtration, the filter cake was washed with ethanol, suction filtered and dried to obtain 83 g of a crystal form of carebastine p-toluenesulfonate with a yield of 83% and a purity of 99.42%.

ESI-MS: m/z = 500.3(M+H) +.

$^1$H NMR (600 MHz, $CDCl_3$) δ: 10.01 (s, 1H), 7.80~7.83 (d, 2H), 7.74~7.76 (d, 2H), 7.43~7.46(d, 2H), 7.27~7.37 (m, 10H), 7.13~7.15 (d, 2H), 5.44 (s, 1H), 3.83~3.85 (m, 1H), 3.53~3.55 (m, 2H), 3.10~3.17 (m, 4H), 3.00~3.05(m, 2H), 2.32(s, 3H), 2.21~2.29 (m, 2H), 2.09~2.19 (m, 2H), 2.01~2.08 (m, 2H), 1.58 (s, 6H).

Results of X-ray powder diffraction, differential scanning calorimetry and thermogravimetric analysis of the crystal form of carebastine p-toluenesulfonate in this example are shown in FIGs. 1 to 3.

**Example 3:** Preparation of a crystal form of carebastine p-toluenesulfonate

500 mg of butanone and 50 mg of crude carebastine p-toluenesulfonate were added at room temperature, and the temperature was raised under stirring to 70-80°C and then kept for 0.2-1.0 h under stirring. The temperature was lowered to 0-15°C, and crystals were precipitated for 0.2-1.2 h. After filtration, the filter cake was washed with butanone, suction filtered and dried to obtain 45 mg of a crystal form of carebastine p-toluenesulfonate with a yield of 90% and a purity of 99.76%. Mass spectrometry, NMR and X-ray powder diffraction results were the same as those in Example 2.

**Example 4:** Preparation of a crystal form of carebastine p-toluenesulfonate

2 g of methyl tert-butyl ether and 100 mg of crude carebastine p-toluenesulfonate were added at room temperature, and the temperature was raised under stirring to 30-55°C and then kept for 0.2-1.0 h under stirring. The temperature was lowered to 0-20°C, and crystals were precipitated for 0.2-1.5 h. After filtration, the filter cake was washed with methyl tert-butyl ether, suction filtered and dried to obtain 95 mg of a crystal form of carebastine p-toluenesulfonate with a yield of 95% and a purity of 99.56%. Mass spectrometry, NMR and X-ray powder diffraction results were the same as those in Example 2.

**Example 5:** Preparation of a crystal form of carebastine p-toluenesulfonate

100 mg of isopropanol, 10 mg of water and 100 mg of crude carebastine p-toluenesulfonate were added at room temperature, and the temperature was raised under stirring to 70-80°C and then kept for 0.2-1.0 h. The temperature was lowered to 0-20°C, and crystals were precipitated for 0.2-1.5 h under stirring. After filtration, the filter cake was washed with isopropanol, suction filtered and dried to obtain 85 mg of a crystal form of carebastine p-toluenesulfonate with a yield of

85% and a purity of 99.86%. Mass spectrometry, NMR and X-ray powder diffraction results were the same as those in Example 2.

**Example 6:** Preparation of a crystal form of carebastine p-toluenesulfonate

16 kg of acetonitrile and 2 kg of crude carebastine p-toluenesulfonate were added at room temperature, and the temperature was raised under stirring to 60-80°C and then kept for 0.5-1.2 h under stirring. The temperature was lowered to 5-20°C, and crystals were precipitated for 0.5-1.5 h. After filtration, the filter cake was washed with acetonitrile, suction filtered and dried to obtain 1.8 kg of a crystal form of carebastine p-toluenesulfonate with a yield of 90% and a purity of 99.66%. Mass spectrometry, NMR and X-ray powder diffraction results were the same as those in Example 2.

**Example 7:** Preparation of a crystal form of carebastine p-toluenesulfonate

1-2.5 kg of methanol and 100 g of crude carebastine p-toluenesulfonate were added at room temperature, and the temperature was raised under stirring to 40-60°C and then kept for 0.5-1.2 h under stirring. The temperature was lowered to 0-15°C, and crystals were precipitated for 0.5-1.5 h. After filtration, the filter cake was washed with methanol, suction filtered and dried to obtain 92 g of a crystal form of carebastine p-toluenesulfonate with a yield of 92% and a purity of 99.86%. Mass spectrometry, NMR and X-ray powder diffraction results were the same as those in Example 2.

**Example 8:** Preparation of a crystal form of carebastine p-toluenesulfonate

0.5-1.5 kg of tetrahydrofuran and 100 g of crude carebastine p-toluenesulfonate were added at room temperature, and the temperature was raised under stirring to 30-60°C and then kept for 0.5-1.2 h under stirring. The temperature was lowered to 0-20°C, and crystals were precipitated for 0.5-1.5 h. After filtration, the filter cake was washed with tetrahydrofuran, suction filtered and dried to obtain 85 g of a crystal form of carebastine p-toluenesulfonate with a yield of 85% and a purity of 99.96%. Mass spectrometry, NMR and X-ray powder diffraction results were the same as those in Example 2.

**Example 9:** Preparation of a crystal form of carebastine p-toluenesulfonate

1.5-2.5 kg of dioxane and 100 g of crude carebastine p-toluenesulfonate were added at room temperature, and the temperature was raised under stirring to 50-80°C and then kept for 0.5-1.2 h under stirring. The temperature was lowered to 0-20°C, and crystals were precipitated for 0.5-1.5 h. After filtration, the filter cake was washed with dioxane, suction filtered and dried to obtain 89 g of a crystal form of carebastine p-toluenesulfonate with a yield of 89% and a purity of 99.66%. Mass spectrometry, NMR and X-ray powder diffraction results were the same as those in Example 2.

**Example 10:** Preparation of a crystal form of carebastine p-toluenesulfonate

1.5-2.5 kg of toluene and 100 g of crude carebastine p-toluenesulfonate were added at room temperature, and the temperature was raised under stirring to 80-100°C and then kept for 0.2-1.5 h under stirring. The temperature was lowered to 0-20°C, and crystals were precipitated for 0.2-1.5 h. After filtration, the filter cake was washed with toluene, suction filtered and dried to obtain 92 g of a crystal form of carebastine p-toluenesulfonate with a yield of 92% and a purity of 99.76%. Mass spectrometry, NMR and X-ray powder diffraction results were the same as those in Example 2.

**Example 11:** Preparation of a crystal form of carebastine p-toluenesulfonate

1.0-2.5 kg of water and 100 g of crude carebastine p-toluenesulfonate were added at room temperature, and the temperature was raised under stirring to 80-100°C and then kept for 0.2-1.5 h under stirring. The temperature was lowered to 0-20°C, and crystals were precipitated for 0.2-1.5 h. After filtration, the filter cake was washed with water, suction filtered and dried to obtain 95 g of a crystal form of carebastine p-toluenesulfonate with a yield of 95% and a purity of 99.66%. Mass spectrometry, NMR and X-ray powder diffraction results were the same as those in Example 2.

**Example 12:** Preparation of a crystal form of carebastine p-toluenesulfonate

0.5-2.5 kg of dimethyl sulfoxide, 0.1-0.5 kg of water and 100 g of crude carebastine p-toluenesulfonate were added at room temperature, and the temperature was raised under stirring to 80-100°C and then kept for 0.2-1.5 h under stirring. The temperature was lowered to 0-20°C, and crystals were precipitated for 0.2-1.5 h. After filtration, the filter cake was washed with water, suction filtered and dried to obtain 95 g of a crystal form of carebastine p-toluenesulfonate with a

yield of 95% and a purity of 99.86%. Mass spectrometry, NMR and X-ray powder diffraction results were the same as those in Example 2.

**Example 13:** Preparation of a crystal form of carebastine p-toluenesulfonate

0.5-2.5 kg of acetonitrile, 0.1-0.5 kg of methanol and 100 g of crude carebastine p-toluenesulfonate were added at room temperature, and the temperature was raised under stirring to 30-60°C and then kept for 0.2-1.5 h under stirring. The temperature was lowered to 0-20°C, and crystals were precipitated for 0.2-1.5 h. After filtration, the filter cake was washed with acetonitrile, suction filtered and dried to obtain 99 g of a crystal form of carebastine p-toluenesulfonate with a yield of 99% and a purity of 99.16%. Mass spectrometry, NMR and X-ray powder diffraction were the same as those in Example 2.

The comparison results of the crystal form structure confirmation data of the carebastine p-toluenesulfonate of Examples 2 to 13 show that they all belong to the same crystal form, with the stacked X-ray powder diffraction pattern of the crystal forms of Examples 2 to 6 shown in FIG. 4. It is demonstrated that the crystal form of the carebastine p-toluenesulfonate of the present invention has good reproducibility, is easy to prepare and suitable for scaled-up production.

**Example X:** Preparation of an amorphous form of carebastine p-toluenesulfonate

1 kg of crude carebastine p-toluenesulfonate was added into 2-4 kg of water, followed by heating and refluxing. After the solid was completely dissolved, the solution was filtered by suction, and the filtrate was spray dried or lyophilized to obtain a white solid. It was shown to be an amorphous form by X-ray powder diffraction as shown in Figure 5 in detail.

ESI-MS: m/z = 500.3$(M+H)^+$.

$^1H$ NMR (600 MHz, $CDCl_3$) δ: 10.01 (s, 1H), 7.80~7.83 (d, 2H), 7.74~7.76 (d, 2H), 7.43~7.46(d, 2H), 7.27~7.37 (m, 10H), 7.13~7.15 (d, 2H), 5.44 (s, 1H), 3.83~3.85 (m, 1H), 3.53~3.55 (m, 2H), 3.10~3.17 (m, 4H), 3.00~3.05(m, 2H), 2.32(s, 3H), 2.21~2.29 (m, 2H), 2.09~2.19 (m, 2H), 2.01~2.08 (m, 2H), 1.58 (s, 6H).

**Test Example 1: Stability study**

**1. Test Samples**

Carebastine (Comparative Example 2), a crystal form of carebastine p-toluenesulfonate (Example 2), and an amorphous form of carebastine p-toluenesulfonate (Example X) were used.

**2. Test method**

3 parts of each of the samples, carebastine (Comparative Example 2), the crystal form of carebastine p-toluenesulfonate (Example 2), and the amorphous form of carebastine p-toluenesulfonate (Example X), were weighed and placed in a weighing bottle, respectively, and each group of samples was placed under conditions of high temperature (60°C), high humidity (RH80%) and light exposure (5000Lux). Then, the samples were taken for detection after 30 days.

Detection method: An appropriate amount of each sample was taken, weighed precisely, dissolved with acetonitrile and quantitatively diluted into a solution containing about 0.08 mg of sample per 1 ml. High performance liquid chromatography (HPLC) was used, with octadecylsilane-bonded silica gel as filler, water-acetonitrile (1:1) as mobile phase, a detection wavelength of 256 nm, a column temperature of 35°C and an injection volume of 10 μl. Each sample solution was injected into the liquid chromatograph instrument, and the chromatograms were recorded. The content of carebastine was calculated by an external standard method, and the impurity content was calculated by a peak area normalization method.

**3. Test results**

After 30 days of tests on influencing factors of high temperature, high humidity and light exposure, the content of carebastine (Comparative Example 2) decreased by more than 1.0% under high temperature and high humidity, and decreased by more than 10% under light exposure, and the content of the maximum single impurity was more than 0.5%; whereas the content of both the crystal form (Example 2) and the amorphous form (Example X) of carebastine p-toluenesulfonate of the present invention decreased by not more than 0.1% under the conditions of high temperature, high humidity, and light exposure, and the content of the maximum single impurity was not more than 0.1%. It is demonstrated that the crystal form and amorphous form of the carebastine p-toluenesulfonate are stable and more suitable for pharmaceutical use.

**Table 1. Test Results of the influencing factors**

| Group | Content | | | |
|---|---|---|---|---|
| | 0 day | High temperature, 30 days | High humidity, 30 days | Light exposure, 30 days |
| Comparative Example 2 | 99.18% | 98.12% | 98.11% | 87.28% |
| Example 2 | 99.42% | 99.43% | 99.42% | 99.39% |
| Example X | 98.59% | 98.54% | 98.58% | 98.51 % |

**Test Example 2: Hygroscopicity study**

**1. Test samples**

Carebastine (Comparative Example 2), carebastine hydrochloride (Comparative Example 3), a crystal form of carebastine p-toluenesulfonate (Example 2), and an amorphous form of carebastine p-toluenesulfonate (Example X) were used, wherein the carebastine hydrochloride was prepared as described in the patent US6340761B1 with a purity of 99% or more.

**2. Test method**

(1) The day prior to the test, a dry glass weighing bottle (with an outer diameter of 50 mm and a height of 15 mm) with a lid was placed in a suitable constant temperature dryer (the lower part of which holds a saturated solution of ammonium chloride or ammonium sulphate) at 25°C ± 1°C or an artificial climate chamber (at a temperature of 25°C ± 1°C and a relative humidity of 80% ± 2%), and the weight (m1) of the bottle with the lid was precisely weighed.

(2) An appropriate amount of the sample to be tested was evenly laid in the weighing bottle, with a thickness of generally about 1 mm, and the weight (m2) of the bottle with the lid was precisely weighed.

(3) The weighing bottle was kept open and placed together with the lid under constant temperature and humidity for 24 h.

(4) The weighing bottle was covered with the lid, and the weight (m3) was precisely weighed.

(5)

$$\text{Percentage of weight gain} = (m3\text{-}m2)/(m2\text{-}m1)\times 100\%.$$

**3. Test results**

The hygroscopicity results are shown in the following table. The results show that the crystal form of carebastine p-toluenesulfonate (Example 2) has little to no hygroscopicity, and the amorphous form of carebastine p-toluenesulfonate (Example X) is slightly hygroscopic. In contrast, the free base of carebastine (Comparative Example 2) is hygroscopic, and the hydrochloride thereof (Comparative Example 3) is extremely hygroscopic. This indicates that the crystal form and the amorphous form of carebastine p-toluenesulfonate of the present invention have better hygroscopic properties, which is significantly superior to the free base of carebastine or the hydrochloride form thereof.

**Table 2. Results of hygroscopicity**

| Compound | Percentage of weight gain (%) | Characteristic description |
|---|---|---|
| Comparative Example 2 | 6 | Hygroscopic |
| Comparative Example 3 | 15.9 | Extremely hygroscopic |
| Example 2 | -0.1 | Little to no hygroscopicity |

(continued)

| Compound | Percentage of weight gain (%) | Characteristic description |
|---|---|---|
| Example X | 0.3 | Slightly hygroscopic |

The above results are determined according to the pharmacopoeia: "hygroscopic" means that the hygroscopic weight gain is less than 15% but not less than 2%; "extremely hygroscopic" means that the hygroscopic weight gain is not less than 15%; "little to no hygroscopicity" means that the hygroscopic weight gain is less than 0.2%; and "slightly hygroscopic" means that the hygroscopic weight gain is less than 2% but not less than 0.2%.

**Test Example 3: Pharmacokinetic Tests**

In this test example, the bioavailability of the crystal form and amorphous form of carebastine p-toluenesulfonate was investigated through pharmacokinetic tests.

**1. Test drugs**

Ebastine (Comparative Example 1), carebastine (Comparative Example 2), a crystal form of carebastine p-toluenesulfonate (Example 2) and an amorphous form of carebastine p-toluenesulfonate (Example X) were prepared into enteric capsules, respectively.

**2. Test method**

(1) Experimental animals

Species: Beagle;

Level: normal level;

Animal number and gender: 8 males;

Age: 1-2 weeks old during grouping;

Body weight: the weight during grouping ranged from 8.90 to 10.3 kg, with an average of 10.0 kg.

(2) Dosage regime and route of administration

Dose: in terms of carebastine, each group was dosed at equimolar dose, and appropriate amounts of compounds were weighed and filled into capsules. That is, the drug of carebastine in each capsule is 2.23 mg.

**Table 3. Design of grouping and administration**

| Cycle | Group | Test subject | Animal number | Administration | |
|---|---|---|---|---|---|
| | | | | Dose (Capsules/Animal) | Route of administration |
| I | 1 | Compound of Comparative Example 1 | 2 | 1 | Intragastric |
| | 2 | Compound of Comparative Example 2 | 2 | 1 | Intragastric |
| | 3 | Compound of Example 2 | 2 | 1 | Intragastric |
| | 4 | Compound of Example X | 2 | 1 | Intragastric |

(continued)

| | | | | | |
|---|---|---|---|---|---|
| 7-day elution period | | | | | |
| II | 1 | Compound of Example X | 2 | 1 | Intragastric |
| | 2 | Compound of Comparative Example 1 | 2 | 1 | Intragastric |
| | 3 | Compound of Comparative Example 2 | 2 | 1 | Intragastric |
| | 4 | Compound of Example 2 | 2 | 1 | Intragastric |
| III | 1 | Compound of Example 2 | 2 | 1 | Intragastric |
| | 2 | Compound of Example X | 2 | 1 | Intragastric |
| | 3 | Compound of Comparative Example 1 | 2 | 1 | Intragastric |
| | 4 | Compound of Comparative Example 2 | 2 | 1 | Intragastric |
| 7-day elution period | | | | | |
| IV | 1 | Compound of Comparative Example 2 | 2 | 1 | Intragastric |
| | 2 | Compound of Example 2 | 2 | 1 | Intragastric |
| | 3 | Compound of Example X | 2 | 1 | Intragastric |
| | 4 | Compound of Comparative Example 1 | 2 | 1 | Intragastric |

After the test compounds were filled into capsules, 2 x 4 crossover tests were carried out according to the table above. A 7-day elution period was counted from the start of administration. The corresponding test compounds were administered to each group in different test cycles.

All animals were fasted overnight (for 12 h or more) before each administration, and feeding was resumed 4 h after the administration. The animals had free access to drinking water.

(3) Plasma sample collection

Blood samples were collected at 0 h before administration, and at 10 min, 20 min, 30 min, 45 min, 1 h, 2 h, 3 h, 5 h, 6 h, 8 h, 10 h and 24 h after administration, for a total of 13 blood collection time points.

Approximately 2 mL of blood was collected from a forelimb vein at the set blood collection time points and placed in an EDTA-K2 anticoagulation tube. Whole blood samples were kept at room temperature for 1.5 h after the collection and then centrifuged (at 3000 g centrifugal force, 4°C) for 10 min.

(4) Biological sample analysis

The concentration of carebastine in plasma was determined using liquid chromatography-mass spectrometry-mass spectrometry (HPLC-MS/MS).

(5) Data acquisition and processing

The pharmacokinetic parameters of each group were calculated using the non-compartmental model with the WinNonlin software (version 8.3 or higher).

**3. Test results**

The results of the pharmacokinetic test of carebastine in the plasma of each beagle group are shown in the following table.

**Table 4. Summary of pharmacokinetic parameters of carebastine in each group (mean ± standard deviation)**

| PK parameters | $T_{max}$ (h) | $T_{1/2}$ (h) | $C_{max}$ (ng/mL) | $AUC_{0-t}$ (h*ng/mL) |
|---|---|---|---|---|
| Comparative Example 1 | 2.25±0.7 | 4.06±1.19 | 179±85.7 | 749±127 |
| Comparative Example 2 | 0.82±0.52 | 3.74±2.02 | 191±51.4 | 840±161 |
| Example 2 | 0.84±0.62 | 5.02±2.02 | 296±44.7 | 1160±252 |

(continued)

| PK parameters | $T_{max}$ (h) | $T_{1/2}$ (h) | $C_{max}$ (ng/mL) | $AUC_{0-t}$ (h*ng/mL) |
|---|---|---|---|---|
| Example X | 0.89±0.59 | 4.35±2.12 | 262±38.9 | 980±261 |

The results above indicate that, compared with the compound of Comparative Example 1, the crystal form of Example 2 and the amorphous form of Example X have significantly shorter peak time ($T_{max}$), with an increase in $C_{max}$ of about 65% and 46% and an increase in $AUC_{0-t}$ of about 55% and 31%, respectively. The half-lives ($T_{1/2}$) are also prolonged respectively.

Compared to the free form of the compound of Comparative Example 2, the crystal form of Example 2 and the amorphous form of Example X have an increase in $C_{max}$ of about 55% and 37%, respectively, and an increase in $AUC_{0-t}$ of about 38% and 17%, respectively. The half-lives ($T_{1/2}$) are also prolonged, respectively, while the $T_{max}$ is essentially the same.

The crystal form of Example 2 has slightly higher $C_{max}$ and $AUC_{0-t}$ than that of the amorphous form of Example X and also a slightly longer $T_{1/2}$.

In conclusion, the pharmacokinetic parameters of the crystal form of Example 2 and the amorphous form of Example X are significantly better than those of the compound of Comparative Example 1 and the compound of Comparative Example 2. This indicates that the crystal form and amorphous form of carebastine p-toluenesulfonate of the present invention allow for rapid uptake into the body to inhibit histamine H1 receptors, and have the potential in clinically treating patients with acute allergic diseases such as acute urticaria, with a stronger and longer-lasting efficacy and better safety profile.

The above examples are only preferred embodiments of the present invention and should not be regarded as limiting the protection scope of the present invention. Any insubstantial changes or modifications made in view of the principal idea and spirit of the present invention that solves the same technical problems as those in the present invention, shall be encompassed in the protection scope of the present invention.

## Claims

1. A crystal form of a compound represented by formula (1),

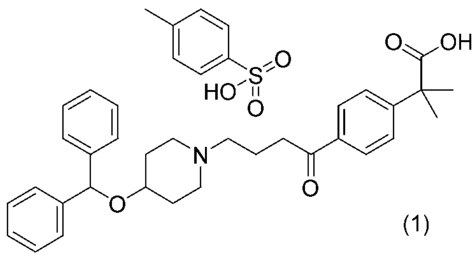

(1)

wherein the crystal form has an X-ray powder diffraction pattern obtained by Cu-Kα radiation that has diffraction peaks at 2θ positions of: 4.26±0.2°, 6.43±0.2°, 14.49±0.2°, 15.11±0.2°, and 18.95±0.2°.

2. The crystal form according to claim 1, wherein the crystal form has an X-ray powder diffraction pattern that has diffraction peaks at 2θ positions of: 4.26±0.2°, 6.43±0.2°, 13.10±0.2°, 14.49±0.2°, 15.11±0.2°, 15.77±0.2°, 18.95 ±0.2°, and optionally 19.12±0.2°.

3. The crystal form according to claim 1 or 2, wherein the crystal form has an X-ray powder diffraction pattern that has diffraction peaks at 2θ positions of: 4.26±0.2°, 6.43±0.2°, 13.10±0.2°, 14.49±0.2°, 15.11±0.2°, 15.77±0.2°, 18.95 ±0.2°, optionally 19.12±0.2°, 19.59±0.2°, 23.49±0.2°, and 24.97±0.2°.

4. The crystal form according to claim 1 or 2, wherein the crystal form has an X-ray powder diffraction pattern with characteristic peaks at the following 2θ positions and corresponding relative intensities:

| 2θ | Relative intensity ($I/I_0$) |
|---|---|
| 4.26±0.2° | 28.8±5% |

(continued)

| 2θ | Relative intensity ($I/I_0$) |
|---|---|
| 6.43±0.2° | 3.7±5% |
| 14.49±0.2° | 65.1±5% |
| 15.11±0.2° | 53.8±5% |
| 18.95±0.2° | 100±5% |
| Optionally 19.12±0.2° | 67.1±5% |

**5.** The crystal form according to any one of claims 1 to 4, wherein the crystal form has an X-ray powder diffraction pattern substantially as shown in FIG. 1.

**6.** The crystal form according to any one of claims 1 to 5, wherein the crystal form has a differential scanning calorimetry thermogram having an endothermic peak at 171°C±5°C;
preferably, the crystal form has a differential scanning calorimetry curve substantially as shown in FIG. 2.

**7.** The crystal form according to any one of claims 1 to 6, wherein the crystal form has a thermogravimetric analysis graph substantially as shown in FIG. 3.

**8.** An amorphous form of a compound represented by formula (1),

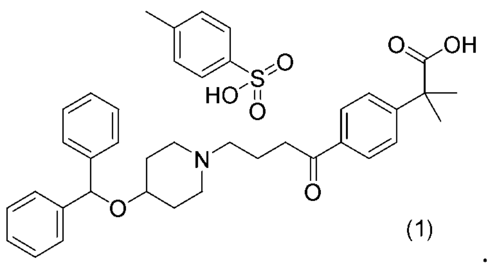

(1)

wherein the amorphous form has an X-ray powder diffraction pattern obtained by Cu-Kα radiation which is represented by a diffuse peak; preferably, the amorphous form has an X-ray powder diffraction pattern substantially as shown in FIG. 5.

**9.** A method for preparing the crystal form according to any one of claims 1 to 7 or the amorphous form according to claim 8, comprising a salt-forming step of:
dissolving an appropriate amount of 2-(4-(4-(4-(diphenylmethoxy)piperidin-1-yl)butyryl)phenyl)-2-methylpropanoic acid and p-toluenesulfonic acid in an organic solvent under stirring and heating to conduct a reaction to form a salt, after completion of the reaction, lowering the temperature to precipitate a solid, and drying the solid to obtain a crude product of carebastine p-toluenesulfonate; preferably, the method for preparing the crystal form comprises the following steps: adding the crude product of carebastine p-toluenesulfonate into a solvent, heating and stirring, then lowering the temperature to precipitate a solid, and suction-filtering and drying the solid to obtain a crystal of carebastine p-toluenesulfonate; and/or the method for preparing the amorphous form comprises the following steps: adding the crude product of carebastine p-toluenesulfonate into a solvent, heating and refluxing, suction-filtering after the solid is completely dissolved, and spray drying or lyophilizing the filtrate to obtain a white solid.

**10.** A pharmaceutical composition comprising the crystal form according to any one of claims 1 to 7 or the amorphous form according to claim 8, wherein the pharmaceutical composition further comprises a pharmaceutically acceptable carrier.

**11.** Use of the crystal form according to any one of claims 1 to 7 or the amorphous form according to claim 8 in the manufacture of a medicament of a histamine H1 receptor antagonist, further comprising a use in the manufacture of a medicament for treating and/or preventing an allergic disease; preferably, the allergic disease is an acute allergic disease; further preferably, the allergic disease is selected from urticaria, allergic rhinitis, eczema, dermatitis, and skin pruritus; more preferably, the acute allergic disease is acute urticaria or acute allergic rhinitis.

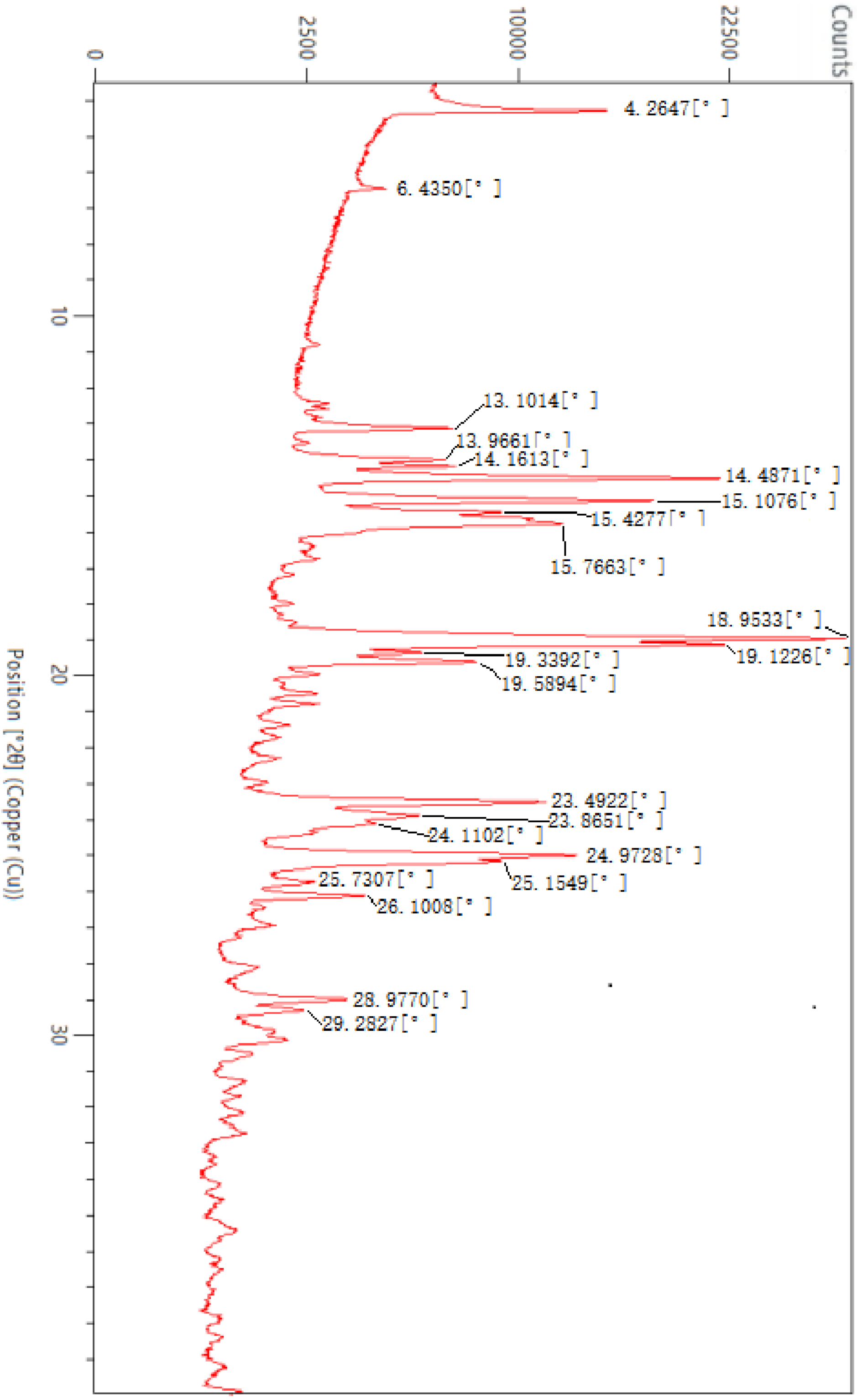
Counts
0
2500
10000
22500
Position [°2θ] (Copper (Cu))
10
20
30
4. 2647[° ]
6. 4350[° ]
13. 1014[° ]
13. 9661[° ]
14. 1613[° ]
14. 4871[° ]
15. 1076[° ]
15. 4277[° ]
15. 7663[° ]
18. 9533[° ]
19. 1226[° ]
19. 3392[° ]
19. 5894[° ]
23. 4922[° ]
23. 8651[° ]
24. 1102[° ]
24. 9728[° ]
25. 1549[° ]
25. 7307[° ]
26. 1008[° ]
28. 9770[° ]
29. 2827[° ]

FIG 1

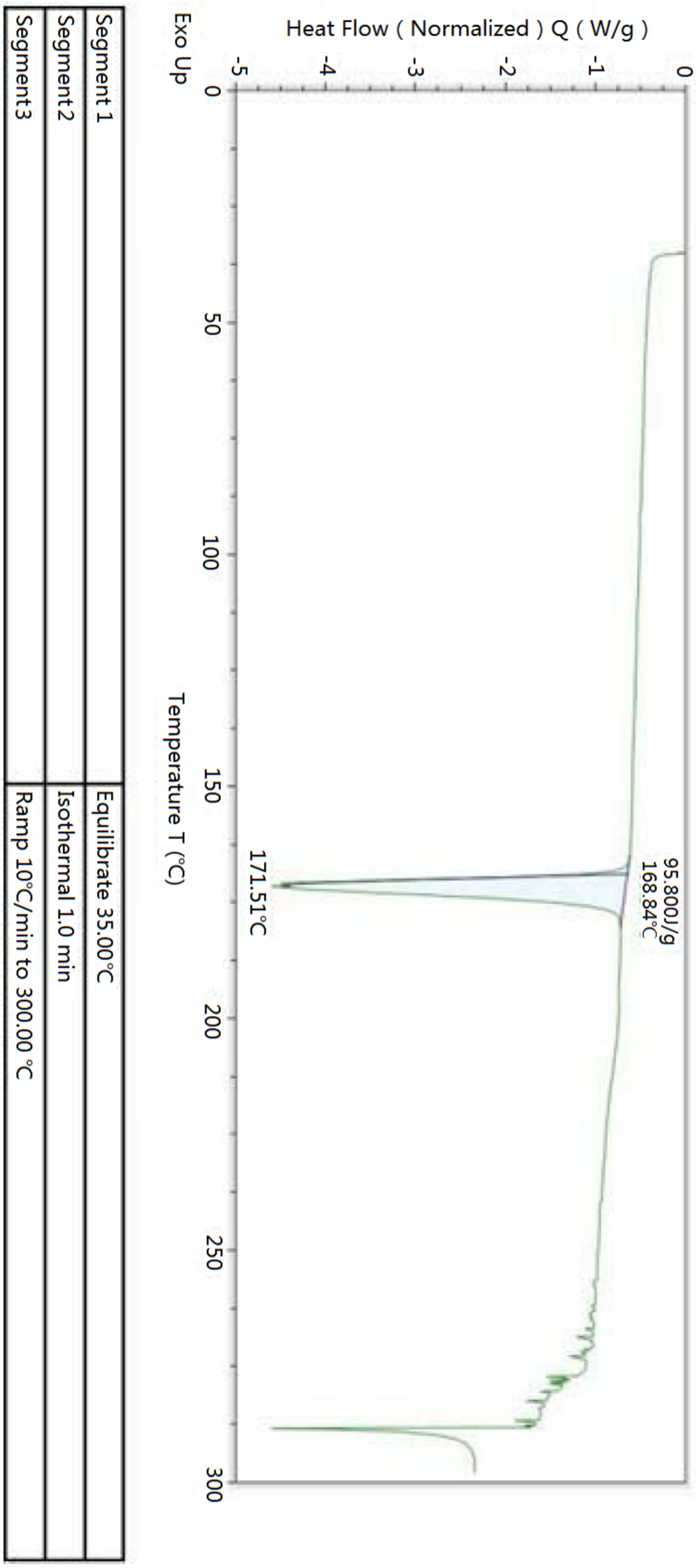
Heat Flow ( Normalized ) Q ( W/g )
Exo Up
Temperature T (°C)
95.800J/g
168.84°C
171.51°C
Segment 1
Equilibrate 35.00°C
Segment2
Isothermal 1.0 min
Segment3
Ramp 10°C/min to 300.00 °C

FIG 2

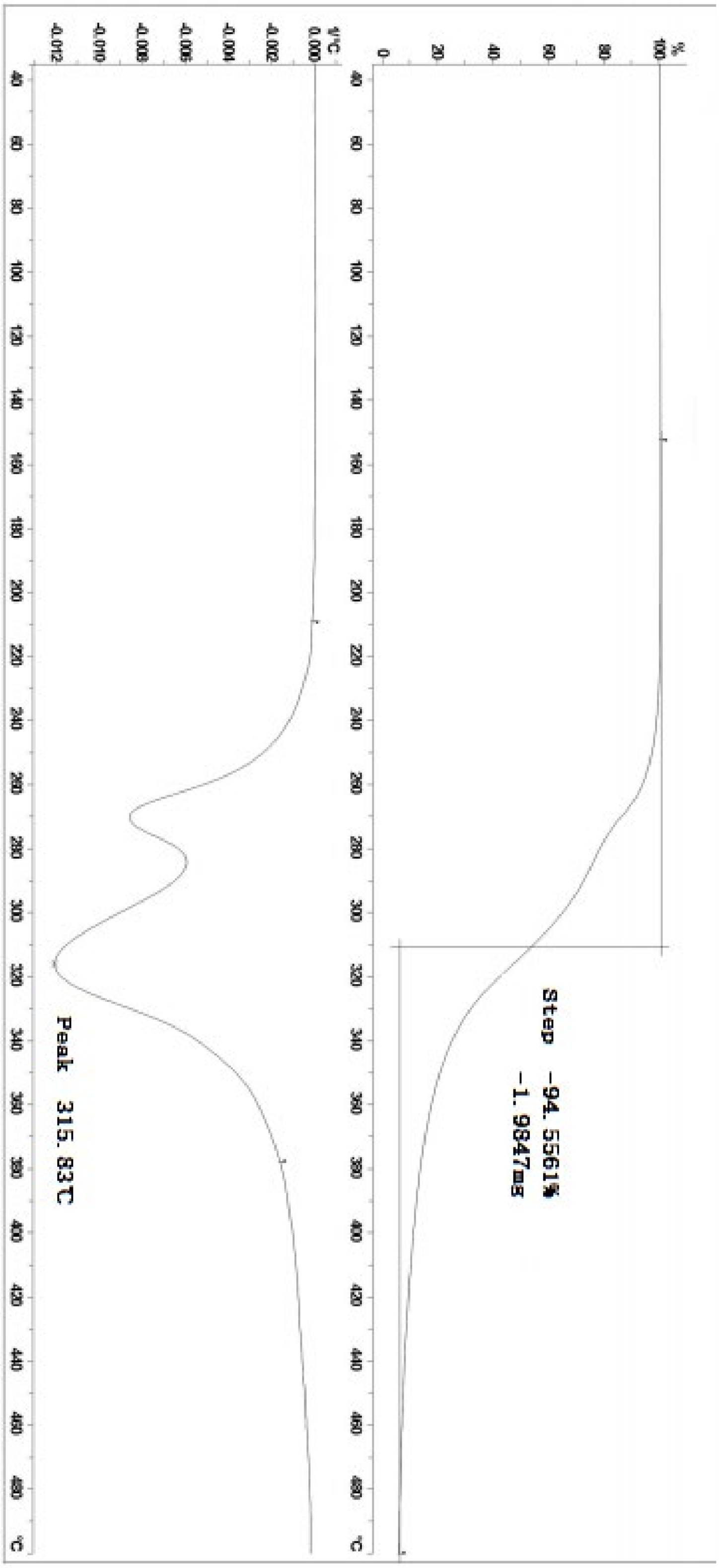


FIG 3

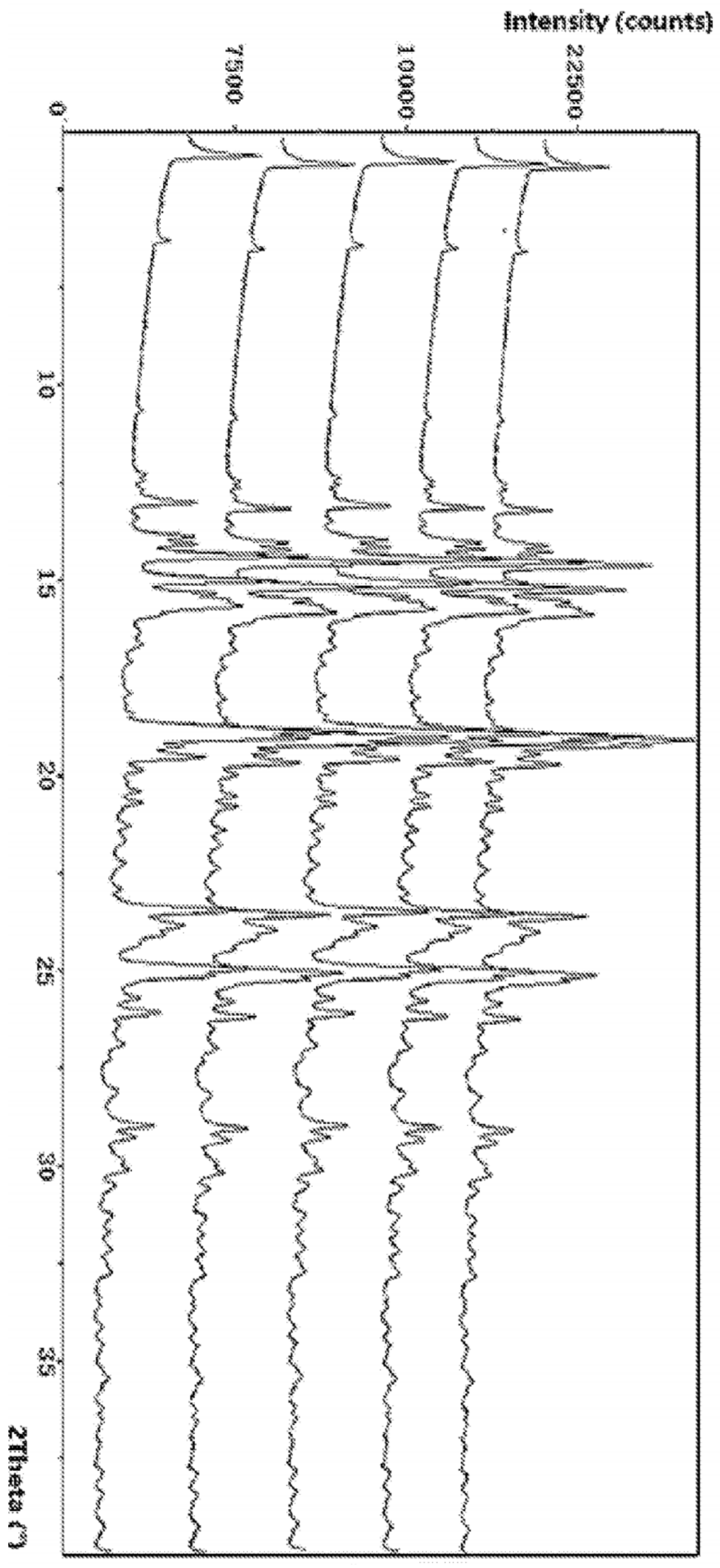


FIG 4

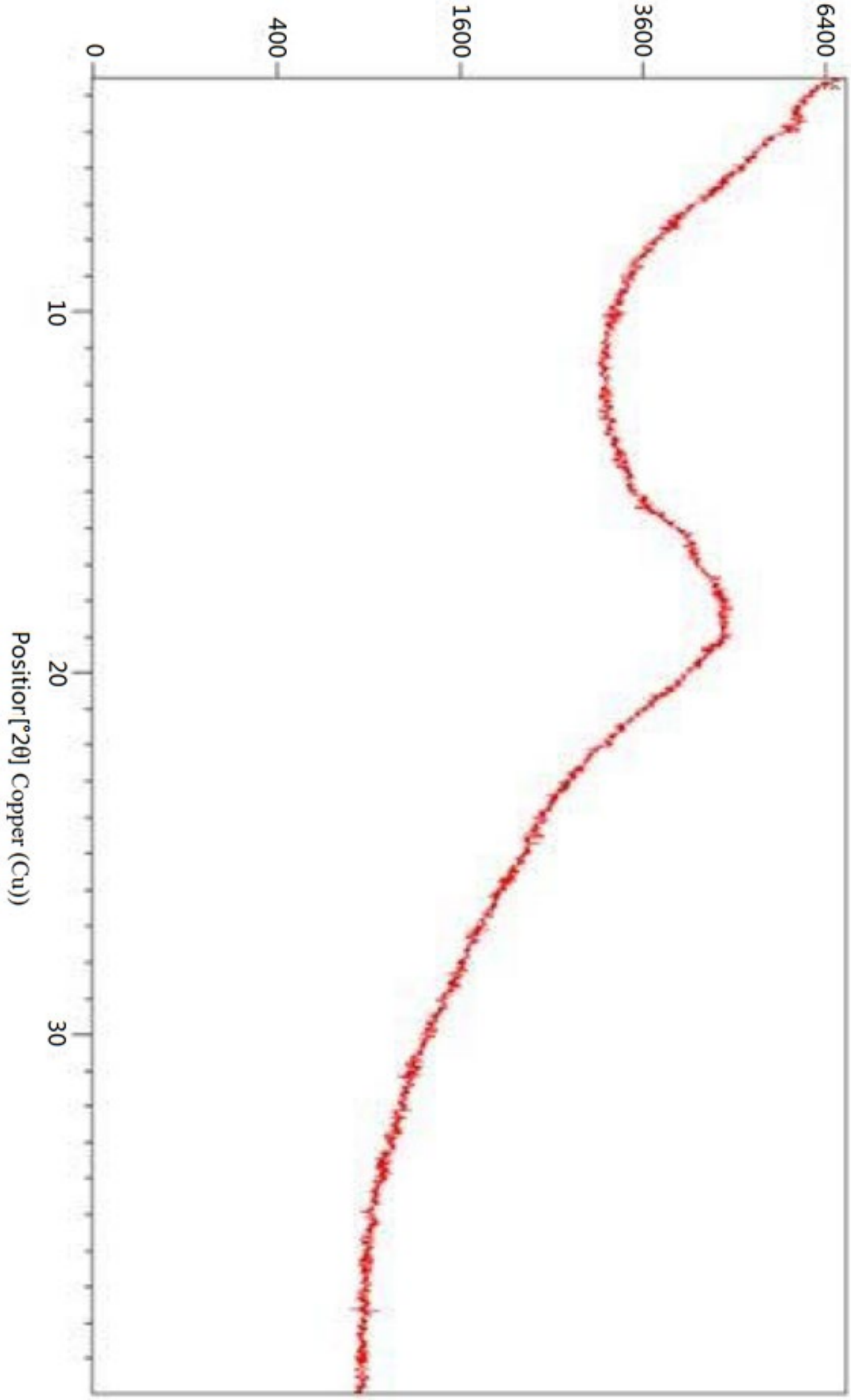


FIG 5

## INTERNATIONAL SEARCH REPORT

International application No.
**PCT/CN2023/088514**

**A. CLASSIFICATION OF SUBJECT MATTER**

C07D211/46(2006.01)i; A61P37/08(2006.01)i; A61P11/02(2006.01)i; A61P17/00(2006.01)i; A61P17/04(2006.01)i; A61K31/445(2006.01)i

According to International Patent Classification (IPC) or to both national classification and IPC

**B. FIELDS SEARCHED**

Minimum documentation searched (classification system followed by classification symbols)

IPC: C07D211/-, A61P37/-, A61P11/-, A61P17/-, A61K31/-

Documentation searched other than minimum documentation to the extent that such documents are included in the fields searched

Electronic data base consulted during the international search (name of data base and, where practicable, search terms used)

CNTXT, ENTXTC, DWPI, VCN, ISI Web of Knowledge, STN(CAPLUS, REGISTRY, MARPAT): 成都施贝康, 哌啶, 卡瑞斯汀, 依巴斯汀, 对甲苯磺酸盐, 组胺, 过敏, 晶体, 无定型, piperidine, carebastine, toluenesulfonic acid, ebastine, histamine, allergy, crystal, amorphous, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| A | WO 2006034091 A2 (AMR TECHNOLOGY, INC. et al.) 30 March 2006 (2006-03-30) embodiment 6 | 1-11 |
| A | WO 9722344 A1 (ALBANY MOLECULAR RESEARCH, INC.) 26 June 1997 (1997-06-26) embodiment 6 | 1-11 |
| A | CN 114014796 A (JIANGSU LIANHUAN PHARMACEUTICAL CO., LTD.) 08 February 2022 (2022-02-08) abstract, and claim 1 | 1-11 |
| A | WO 2011121099 A2 (AREVIPHARMA GMBH et al.) 06 October 2011 (2011-10-06) claim 1, and description, page 10, lines 13-27, and page 22, line 5-page 23, line 5 | 1-11 |
| A | US 4254130 A (RICHARDSON-MERRELL, INC.) 03 March 1981 (1981-03-03) abstract, and claim 1 | 1-11 |
| A | CN 114890994 A (CHENGDU SCHMIDBEKANG BIOMEDICAL TECHNOLOGY CO., LTD.) 12 August 2022 (2022-08-12) abstract, and claim 1 | 1-11 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 August 2023** | **31 August 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/088514**

| Patent document cited in search report | Publication date (day/month/year) | Patent family member(s) | Publication date (day/month/year) |
|---|---|---|---|
| WO 2006034091 A2 | 30 March 2006 | WO 2006034091 A3 | 03 August 2006 |
| | | US 2011065923 A1 | 17 March 2011 |
| | | US 8067604 B2 | 29 November 2011 |
| | | US 2009137813 A1 | 28 May 2009 |
| | | US 7858798 B2 | 28 December 2010 |
| | | US 2006063933 A1 | 23 March 2006 |
| | | US 7498443 B2 | 03 March 2009 |
| WO 9722344 A1 | 26 June 1997 | EP 1362849 A2 | 19 November 2003 |
| | | EP 1362849 A3 | 26 November 2003 |
| | | NO 982806 D0 | 18 June 1998 |
| | | NO 982806 L | 12 August 1998 |
| | | NO 311718 B1 | 14 January 2002 |
| | | DK 0877733 T3 | 11 December 2006 |
| | | NZ 326228 A | 28 February 2000 |
| | | ES 2268715 T3 | 16 March 2007 |
| | | US 6452011 B1 | 17 September 2002 |
| | | AT 335726 T | 15 September 2006 |
| | | US 2005272771 A1 | 08 December 2005 |
| | | US 7560561 B2 | 14 July 2009 |
| | | JP 2000502105 A | 22 February 2000 |
| | | JP 4865113 B2 | 01 February 2012 |
| | | PT 877733 E | 29 December 2006 |
| | | US 2011295014 A1 | 01 December 2011 |
| | | HU 9900947 A2 | 28 July 1999 |
| | | HU 9900947 A3 | 28 December 2000 |
| | | US 2010010227 A1 | 14 January 2010 |
| | | US 8022220 B2 | 20 September 2011 |
| | | KR 20000064507 A | 06 November 2000 |
| | | KR 100516869 B1 | 25 November 2005 |
| | | JP 2009019059 A | 29 January 2009 |
| | | KR 20050086753 A | 30 August 2005 |
| | | KR 100591212 B1 | 22 June 2006 |
| | | CA 2240735 A1 | 26 June 1997 |
| | | CA 2240735 C | 29 August 2006 |
| | | US 6201124 B1 | 13 March 2001 |
| | | BR 9612099 A | 28 December 1999 |
| | | AU 1354797 A | 14 July 1997 |
| | | AU 723759 B2 | 07 September 2000 |
| | | EP 0877733 A1 | 18 November 1998 |
| | | EP 0877733 A4 | 31 March 1999 |
| | | EP 0877733 B1 | 09 August 2006 |
| | | DE 69636439 D1 | 21 September 2006 |
| | | DE 69636439 T2 | 29 March 2007 |
| | | US 2003171590 A1 | 11 September 2003 |
| | | US 6974872 B2 | 13 December 2005 |
| | | US 6448406 B1 | 10 September 2002 |
| CN 114014796 A | 08 February 2022 | None | |
| WO 2011121099 A2 | 06 October 2011 | EP 2371817 A1 | 05 October 2011 |
| | | DE 112011101112 T5 | 10 January 2013 |
| | | WO 2011121099 A3 | 08 March 2012 |

Form PCT/ISA/210 (patent family annex) (July 2022)

**INTERNATIONAL SEARCH REPORT**
**Information on patent family members**

International application No.
**PCT/CN2023/088514**

| Patent document cited in search report | | | Publication date (day/month/year) | Patent family member(s) | | | Publication date (day/month/year) |
|---|---|---|---|---|---|---|---|
| US | 4254130 | A | 03 March 1981 | NL | 8000762 | A | 14 October 1980 |
| | | | | NL | 190664 | B | 17 January 1994 |
| | | | | NL | 190664 | C | 16 June 1994 |
| | | | | BE | 882704 | A | 31 July 1980 |
| | | | | ZA | 80331 | B | 28 January 1981 |
| | | | | AU | 5501880 | A | 16 October 1980 |
| | | | | AU | 536463 | B2 | 10 May 1984 |
| | | | | IT | 8047919 | D0 | 15 February 1980 |
| | | | | IT | 1144079 | B | 29 October 1986 |
| | | | | JPS | 55139360 | A | 31 October 1980 |
| | | | | CA | 1123439 | A | 11 May 1982 |
| | | | | IL | 59157 | A0 | 30 May 1980 |
| | | | | IL | 59157 | A | 29 February 1984 |
| | | | | FR | 2453853 | A1 | 07 November 1980 |
| | | | | FR | 2453853 | B1 | 09 July 1982 |
| | | | | DK | 133080 | A | 11 October 1980 |
| | | | | DK | 160250 | B | 18 February 1991 |
| | | | | DK | 160250 | C | 22 July 1991 |
| | | | | SE | 8002635 | L | 11 October 1980 |
| | | | | SE | 448727 | B | 16 March 1987 |
| | | | | ATA | 126080 | A | 15 March 1984 |
| | | | | AT | 376207 | B | 25 October 1984 |
| | | | | ES | 8103734 | A1 | 16 March 1981 |
| | | | | CH | 648019 | A5 | 28 February 1985 |
| | | | | NZ | 192615 | A | 23 February 1982 |
| | | | | NO | 801015 | L | 13 October 1980 |
| | | | | NO | 154964 | B | 13 October 1986 |
| | | | | NO | 154964 | C | 21 January 1987 |
| | | | | DE | 3005948 | A1 | 30 October 1980 |
| | | | | DE | 3005948 | C2 | 05 October 1989 |
| CN | 114890994 | A | 12 August 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310000722
- US 6340761 B1